# EUROPEAN PATENT SPECIFICATION

(11) **EP 1 814 589 B1**
(45) Date of publication and mention of the grant of the patent: **15.04.2015**
(21) Application number: 05811167.5
(22) Date of filing: 16.11.2005
(51) Int. Cl.: A61K 31/138, A61K 31/58, A61P 25/24, A61P 25/22

(54) **DRUG COMBINATION COMPRISING A SELECTIVE SEROTONIN REUPTAKE INHIBITOR AND A GLUCOCORTICOID RECEPTOR ANTAGONIST FOR THE TREATMENT OF DEPRESSION**
ARZNEISTOFFKOMBINATION ENTHALTEND EINEN SELEKTIVEN INHIBITOR DER SEROTONINWIEDERAUFNAHME UND EINEN GLUCOCORTICOID REZEPTOR ANTAGONIST ZUR BEHANDLUNG VON DEPRESSIONEN
COMBINAISON DE MÉDICAMENTS COMPRENANT D'UN INHIBITEUR SÉLECTIF DE RECAPTAGE DE SÉROTONINE ET D'UN ANTAGONISTE DU RÉCEPTEUR DE GLUCOCORTICOIDES DESTINÉE AU TRAITEMENT DE LA DÉPRESSION

(30) Priority: 19.11.2004 EP 04257199
(43) Date of publication of application: 08.08.2007
(60) Divisional application: 10179145.7
(73) Proprietor: MSD Oss B.V., 5349 AB Oss (NL)
(72) Inventor: INGRAM, Collin D., Newcastle upon Tyne NE2 4HH (GB); GRASSIE, Morag A., Newhouse, Lanarkshire Scotland ML1 5SH (GB); FERRIER, Nicol, Newcastle upon Tyne NE1 4LP (GB); GARTSIDE, Sasha E., Newcastle upon Tyne NE2 4HH (GB); YOUNG, Allan H., Newcastle upon Tyme NE1 4LP (GB)
(74) Representative: Hussain, Deeba
(86) International application number: PCT/EP2005/056023
(87) International publication number: WO 2006/053884

(56) References cited:
- WO-A-03/037354
- WO-A-2004/030618
- YOUNG ELIZABETH A ET AL: "HPA axis activation in major depression and response to fluoxetine: a pilot study." PSYCHONEUROENDOCRINOLOGY. OCT 2004, vol. 29, no. 9, October 2004 (2004-10), pages 1198-1204, XP002325433 ISSN: 0306-4530 cited in the application
- SPINKS D ET AL: "Serotonin reuptake inhibition: an update on current research strategies.", CURRENT MEDICINAL CHEMISTRY APR 2002, vol. 9, no. 8, April 2002 (2002-04), pages 799-810, XP055096471, ISSN: 0929-8673
- LE POUL E ET AL: "Fluoxetine-induced desensitization of somatodendritic 5-HT1A autoreceptors is independent of glucocorticoid(s).", SYNAPSE (NEW YORK, N.Y.) DEC 1997, vol. 27, no. 4, December 1997 (1997-12), pages 303-312, XP055090167, ISSN: 0887-4476, DOI: http://onlinelibrary.wiley.com/store/10.10 02/(SICI)1098-2396(199712)27:4

## Description

The invention relates to a drug combination for the treatment of depression and related disorders comprising a specific serotonin reuptake inhibitor.

A recent clinical study has demonstrated that in patients with hypothalamic-pituitary-adrenal (HPA) axis disturbances, the anti-depressant efficacy of a selective serotonin reuptake inhibitor (SSRI) was less in patients with high stress hormone levels (Young et al.; HPA axis activation in major depression and response to fluoxetine: a pilot study, Psychoneuroendocrinology, 2004, vol 29, pp 1198-1204). It was suggested that those patients might be treated with antidepressants other than SSRIs.

Other disclosures discuss the role of HPA function in psychopathology or mechanism of action of SSRIs or suggest the use of glucocorticoid receptor antagonists (GR-antagonists) for treatment in patients with overactive stress hormone functions (Blackburn-Munro and Blackburn-Munro, Chronic pain, chronic stress and depression: coincidence or consequence? J. Neuroendocrinology, 2001, Vol 13, pp 1009-1023; Strohle and Holsboer, Stress responsive neurohormones in depression and anxiety, Pharmacopsychiatry 2003, vol 36, suppl 3 pp S207-S214; Van Praag, Can stress cause depression? 2004, Progress in Neuro-Psychopharmacol. & Biol. Psychiatry, 2004, Vol 28, pp 891-907; Le Poul et al., Fluoxetine-induced desensitization of somatodendritic 5-HT1A autorecptors is independent of glucocorticoid(s), Synapse, 1997, vol 27, pp 303-312).

This invention provides a GR antagonist or a pharmaceutically acceptable salt thereof for use in the treatment of depression or a related disorder in a patient to whom an SSRI or a pharmaceutically acceptable salt thereof is also administered;
characterized in that the GR antagonist is (11β, 17β)-11-(1,3-benzodioxol-5-yl)- 17-hydroxy-17-(1-propynyl)-estra-4,9-dien-3-one and the SSRI is fluoxetine or a pharmaceutically acceptable salt thereof, and wherein the depression related disorder is selected from anxiety disorder, panic disorder, obsessive compulsive disorder, post-traumatic stress disorder or bipolar disorder.

The combination has better effects in more patients in comparison to each drug alone. The better effect can reside in less side effects or a faster (early or accelerated onset) or more complete recovery in individual patients or in the overall result of the treatment of a group of patients. The preferred use of the combination will be in the treatment of treatment-resistant depression, also known as refractory depression or treatment refractory depression.

The following specifications of the terms used above serve to clarify better what is provided by this invention.

An SSRI is a drug having selective serotonin reuptake inhibiting effect. This means that it is at least twice more active in inhibiting the serotonin transporter than the noradrenaline transporter and the dopamine transporter. The inhibition of the transporters is measurable by many known techniques. Known SSRIs are citalopram, S-citalopram, clomipramine, fluoxetine, fluvoxamine, paroxetine and sertraline. A GR antagonist is a drug that blocks the action of cortisol and corticosterone on the glucocorticosteroid receptor. Known examples of GR antagonists are RU 486 (RU 38486 = (11β.17β)-11-[4-(dimethylamino)phenyl]-17-hydroxy-17-(1-propynyl)-estra-4,9-dien-3-one), Org 34517, which is (11β,17β)-11-(1,3-benzodioxol-5-yl)-17-hydroxy-17-(1-propynyl)-estra-4,9-dien-3-one, Org 34850 which is (11β,17α)-11-[4-(dimethylamino)phenyl-17-hydroxy-21-[4-(methylsulfonyl)phenyl-19-norpregna-4,9-dien-20-yn-3-one and ketoconazole. Org 34517 is the GR antagonist and fluoxetine is the SSRI for use according to this invention.

Unless otherwise stated all amounts of the active components refer to the weights of the compounds as base or acid and not to the weight of the salt. According to the terminology in this description the drugs referred to as an SSRI or a GR antagonist are the active ingredients or active components of the combination, namely fluoxetine or Org 34517 repectively.

Pharmaceutically acceptable salts include acid addition salts, for example, hydrochloric, fumaric, maleic, citric or succinic acid, these acids being mentioned only by way of illustration and without implied limitation.

The terms pharmaceutically acceptable carriers and excipients refer to those substances known in the art to be allowable as filler or carrier material in pills, tablets, capsules etc. The substances are usually approved for this purpose by health-care authorities and are inactive as pharmacological agents. A compilation of pharmaceutically acceptable carriers and excipients can be found in the Handbook of Pharmaceutical excipients (2nd edition edited by A. Wade and P.J. Weller; Published by the American Pharmaceutical Association, Washington and The Pharmaceutical Press, London in 1994). Specifically, lactose, starch, cellulose derivatives and the like, or mixtures thereof, can be used as carriers for the active components of the combination according to this invention. Accessory ingredients include those conventional in the art, such as colorants, binders, diluents, disintegrants, lubricants, flavouring agents and wetting agents. In general any pharmaceutically acceptable additive which does not interfere with the function of the active compounds can be used.

The term combination refers to any presentation form in which the intention for combined use of the SSRI and the GR antagonist can be recognized.

Such combinations of the SSRI and the GR antagonist may in this description also be referred to as combinations according to the invention.
It will be appreciated that the compounds of the combination may be administered concomitantly, either in the same or different pharmaceutical formulation or sequentially. If there is sequential administration, the delay in administering the second (or additional) active ingredient should not be such as to lose the benefit of the efficacious effect of the combination of the active ingredients. A minimum requirement for a combination according to this description is that the combination should be intended for combined use with the benefit of the efficacious effect of the combination of the active ingredients. The intended use of a combination can be inferred by facilities, provisions, adaptations and/or other means to help using the combination according to the invention. For example, a combination can be made suitable by adding instructions or aids or even determinants for the combined use. Determinants for the combined use can, for example, reside in the properties of a dispenser of dosage units of the active ingredients of the combination. The active ingredients can thus be in separate dosage units, but still the combination can have a determinant inducing the use of the dosage units of the combination in a predetermined sequence and/or at pre-determined times by the properties of the dispenser. A preferred determinant for combined use is of course the formulation of both the active components of the combination in one pharmaceutical composition.
Thus according to one aspect, the present invention provides a pharmaceutical composition, comprising the SSRI, or a pharmaceutically acceptable salt or solvate thereof, and the GR antagonist, or a pharmaceutically acceptable salt or solvate thereof.

The term 'depression and related disorders' refers to a medical field which can be understood by the skilled practitioner. Those disorders known to respond positively to treatment with drugs classified as SSRIs are considered for the description of this invention as being related to depression. Such disorders are for example anxiety disorders, such as panic disorder, obsessive compulsive disorder, posttraumatic stress disorder, chronic pain syndromes or bipolar disorder. It is well known that SSRIs have more general beneficial effects on behaviour and mental functioning which is not strictly limited to an effect on depression. Also included in the invention is the use of the combination in anxiety in the manner in which SSRIs are used, that is with extended use for a long term effect, which is to be distinguished from the use of typical anxiolytic drugs, also referred to as minor tranquillizers, which have an acute anxiety relieving and often sedative effect. The latter anxiolytic/sedative effect is usually ascribed to interactions with the GABA-receptor in the brain.

While it is possible for the active ingredients of the combination to be administered as the pure chemical it is preferable to present them as a pharmaceutical composition, also referred to in this context as pharmaceutical formulation. Suitable compositions include those suitable for oral or rectal administration. Pharmaceutical compositions in embodiments of the present invention comprise the SSRI or the GR antagonist or a combination thereof together with one or more pharmaceutically acceptable carriers or excipients and optionally other therapeutic agents. The present invention further provides compositions according to the invention for use in therapy of depression and related disorders. Furthermore, the invention includes the use of the SSRI or the GR antagonist in the manufacture of a medicament comprising the SSRI or the GR antagonist, having psychotropic activity with improved efficacy for therapy, in particular, of depression and related disorders. This medicament has an enhanced effect or less side effects or an earlier onset of action in comparison to each drug alone. The preferred use of the medicament will be for the treatment of treatment-resistant depression. The invention includes as well the use of the SSRI or the GR antagonist in the manufacture of medicaments for administration in combination (either concomitantly or sequentially) with the GR antagonist or the SSRI, respectively, for the treatment of depression or related disorders.

An important utility of the present invention is a method for the treatment of an individual of a vertebrate species, for example, a mammal including a human patient, suffering from depression or a related disorder, which method of treatment comprises administering an effective amount of the SSRI in combination with the GR antagonist. The desired daily doses for a treatment is preferably presented as a single dose or in two, or three sub-doses administered at appropriate intervals throughout the day. In practice this means among others to provide dosage units comprising the SSRI and dosage units comprising the GR antagonist in a combination or to provide dosage units comprising the SSRI and the GR antagonist for administration to a recipient or intake by a recipient for treatment.
Thus, in one embodiment of the invention a mixture of the SSRI and the GR antagonist may be presented as a pharmaceutical formulation in unit dosage form, for example, administered in the form of a tablet, pill, capsule and the like. Such dosage forms and their preparations are known in the art, e.g. as described in the standard reference, Gennaro et al., Remmington: The science and practice of pharmacy (20th ed., Lippincott Williams & Wilkins, Baltimore, Philadelphia, 2000, see especially Part 5: Pharmaceutical manufacturing). Suitable auxiliaries are described in e.g. the Handbook of Pharmaceutical Excipients (2nd Edition, Editors A. Wade and P.J. Weller; American Pharmaceutical Association; Washington; The Pharmaceutical Press; London, 1994). Such methods include the step of bringing into association an active ingredient with a carrier which constitutes one or more accessory ingredients. The invention provides therefore also the process of preparation of pharmaceutical compositions and more specifically dosing units comprising the SSRI and the GR antagonist, which process comprises bringing an amount of the SSRI (or a pharmaceutically acceptable salt thereof) and amount of the GR antagonist (or a pharmaceutically acceptable salt thereof) into association with one or more pharmaceutical excipients.

More commonly these days pharmaceutical formulations are prescribed to the patient in "patient packs" containing a number dosing units or other means for administration of metered unit doses for use during a distinct treatment period in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical formulation, as herein before described, in combination with packaging material suitable for said formulations. In such a patient pack the intended use of a formulation for the combination treatment of depression or related disorders can be inferred by instructions, facilities, provisions, adaptations and/or other means to help using the formulation most suitably for the treatment. Such measures make a patient pack specifically suitable for and adapted for use for treatment with the combination of the present invention.

Specifically, a further embodiment includes a package containing separate dosage units, one or more of which contains the SSRI or a pharmaceutically acceptable salt thereof and one or more of which contains the GR antagonist or a pharmaceutically acceptable salt thereof. A package contains enough tablets, capsules or the like to treat a patient for a pre-determined period of time, for instance for 2 weeks, 1 month or 3 months.

For the use of the combination of the present invention it should provide the active ingredients such that effective amounts for treatment are made available. The amount of a combination of the SSRI (or a pharmaceutically acceptable salt or solvate thereof) and the GR antagonist (or a pharmaceutically acceptable salt or solvate thereof), required to produce the efficacious effects will, of course, vary and is ultimately at the discretion of the medical practitioner. The factors to be considered include the route of administration and nature of the formulation, the recipient's body weight, age and general condition and the nature and severity of the disease to be treated. In general, a suitable dose of the SSRI for administration to a human will be in the range of 0.05 to 5 mg per kilogram body weight of the recipient per day, preferably in the range of 0.1 to 1.0 mg per kilogram body weight per day.
A suitable dose of the GR antagonist for administration to a human will usually be in the range of from 0.01 to 5 mg per kilogram body weight of the recipient per day, preferably in the range of from 0.05 to 0.7 mg per kilogram body weight per day.

Formulations suitable for oral administration may be presented as discrete units such as tablets or capsules each containing a predetermined amount of active ingredient(s). Suitable amounts of active ingredients in a dosing unit are, for example, a tablet comprising 1 to 50 mg of the SSRI and typically 5 to 100 mg of the GR antagonist.

### Example

Desensitization of the 5-HT_{1A} autoreceptor present on raphe neurones is considered to be one of the important mechanisms underlying the action of SSRIs. In experimental animals this desensitization can be measured using microdialysis in the prefrontal cortex and a combination of local administration of an SSRI (fluoxetine) in the region of the probe to block local uptake and systemic injection which affects all uptake sites and leads to 5-HT_{1A} autoreceptor activation.

### METHOD

Studies were performed on Hooded Lister rats housed in groups of three with food and water available ad libitum. Animals were kept in controlled environmental conditions: temperature (20°C) and humidity (30-50%) on a 12 h light/dark cycle (lights on 07.00 a.m.). Each cage received the same treatment and animals from a single cage studied on the same day to avoid social isolation. Animals underwent two treatments; vehicle or GR antagonist (15 mg/kg Org 34850, intraperitoneally (i.p.)) given as twice daily injections at 07:00-07:30 and 19:00-19:30. All animals received a single daily injection of fluoxetine (10 mg/kg in sterile water i.p.) given in the morning. Treatment periods were 0 (control), 3, 7 or 14 days.

For microdialysis animals were anaesthetised with chloral hydrate (500 mg/kg i.p. plus supplementary doses as required). Surgery was undertaken to position a microdialysis probe in the right prefrontal cortex (stereotaxic coordinates: rostral +3.2 mm, lateral 0.5 mm, ventral -4.6 mm from bregma and dura surface). The probe was perfused with artificial cerebrospinal fluid (aCSF) for 3 h until stable basal level of 5-HT is achieved. Samples were collected every 20 minutes and analysed by HPLC with electrochemical detection. The experimental protocol comprised three phases: i) Basal extracellular levels of 5-HT were measured for 60 min.; ii) Fluoxetine (10 µM) was added to the aCSF to block local uptake in order to measure total release rate. Samples were collected for 80 min. and iii) Fluoxetine was injected i.p. (10 mg/kg) to block all uptake sites, including at the autoreceptor, and samples were collected for 120 min. The magnitude of the fall in measured 5-HT levels after this injection was used as a measure of autoreceptor sensitivity.

### RESULTS

**Table: Effect of pretreatment with fluoxetine or the combination of fluoxetine and Org 34850 given for 3, 7 or 14 days on the fall in 5-HT levels following an i.p. injection of fluoxetine.**

| | Mean Fall in 5-HT levels (fmol) | |
|---|---|---|
| No pretreatment (control) | 13.00 | |
| | Pretreated with fluoxetine and vehicle | Pretreated with fluoxetine and Org 34850 |
| Pretreatment number of days: | | |
| 3 days | 7.59 | 6.46 |
| 7 days | 7.37 | 1.68 |
| 14 days | 4.85 | 2.55 |

The results, as illustrated in the Table, show that the magnitude of the fall was less in the groups receiving the combination treatment compared to fluoxetine alone.

### CONCLUSION

The results indicate that, following combination treatment with SSRI and GR antagonist, the autoreceptor has undergone a greater desensitization compared to the SSRI alone.

## Claims

1. A GR antagonist or a pharmaceutically acceptable salt thereof for use in the treatment of depression or a related disorder in a patient to whom an SSRI or a pharmaceutically acceptable salt thereof is also administered;
**characterized in that** the GR antagonist is (11β,17β)-11-(1,3-benzodioxol-5-yl)-17-hydroxy-17-(1-propynyl)-estra-4,9-dien-3-one and the SSRI is fluoxetine or a pharmaceutically acceptable salt thereof,
and wherein the depression related disorder is selected from anxiety disorder, panic disorder, obsessive compulsive disorder, post-traumatic stress disorder or bipolar disorder.

## Patentansprüche

1. Ein GR-Antagonist oder ein pharmazeutisch annehmbares Salz davon zur Verwendung bei der Behandlung einer Depression oder einer damit verbundenen Störung bei einem Patienten, dem auch ein SSRI oder ein pharmazeutisch annehmbares Salz davon verabreicht wird,
**dadurch gekennzeichnet, dass** der GR-Antagonist (11β, 17β)-11-(1,3-Benzodioxol-5-yl)-17-hydroxy-17-(1-propinyl)-estra-4,9-dien-3-on ist und der SSRI Fluoxetin oder ein pharmazeutisch annehmbares Salz davon ist,
und wobei die mit Depression verbundene Störung ausgewählt ist aus Angststörung, Panikstörung, Zwangsstörung, posttraumatischer Stressstörung oder bipolarer Störung.

## Revendications

1. Antagoniste de GR ou un sel pharmaceutiquement acceptable de celui-ci pour une utilisation dans le traitement d'une dépression ou d'un trouble apparenté chez un patient à qui un SSRI ou un sel pharmaceutiquement acceptable de celui-ci est aussi administré;
**caractérisé en ce que** l'antagoniste de GR est la (11β,17β)-11-(1,3-benzodioxol-5-yl)-17-hydroxy-17-(1-propynyl)-estra-4,9-dién-3-one et le SSRI est la fluoxétine ou un sel pharmaceutiquement acceptable de celle-ci,
et où le trouble apparenté à une dépression est choisi parmi un trouble de l'anxiété, un trouble de panique, un trouble obsessionnel compulsif, un trouble de stress post-traumatique ou un trouble bipolaire.
